# EUROPEAN PATENT APPLICATION

(11) **EP 3 964 119 A1**
(43) Date of publication of application: **09.03.2022**
(21) Application number: 21194108.3
(22) Date of filing: 31.08.2021
(51) Int. Cl.: A61B 3/10

(54) **OPHTHALMIC DEVICE**

(30) Priority: 02.09.2020 JP 2020147803
(71) Applicant: Tomey Corporation, Nagoya-shi, Aichi 451-0051 (JP)
(72) Inventor: OKAMOTO, Keiichiro, Nagoya-shi, 4510051 (JP)
(74) Representative: Winter, Brandl - Partnerschaft mbB

(57) **Abstract**

An ophthalmic device may be provided with: a first scanning optical system configured to scan first light outputted from a first light source (26) in a first range of a subject eye (E); and a second scanning optical system configured to scan a second light outputted from a second light source (76) in a second range of the subject eye (E) that is different from the first range. A central wavelength of the first light and a central wavelength of the second light are different. The first scanning optical system includes a first objective lens unit (42) configured to irradiate the first light to the first range of the subject eye (E). The second scanning optical system includes a second objective lens unit (54) configured to irradiate the second light to the second range of the subject eye (E).

## Description

### CROSS-REFERENCE TO RELATED APPLICATION

This application claims priority to Japanese Patent Application No. 2020-147803, filed on September 2, 2020, the entire contents of which are incorporated herein by reference.

### TECHNICAL FIELD

The art disclosed herein relates to an ophthalmic device. More specifically, the art disclosed herein relates to an ophthalmic device configured to acquire tomographic information for each of different measurement ranges (such as an anterior segment and a fundus) of a subject eye.

### BACKGROUND

An ophthalmic device that acquires tomographic information for each of different measurement ranges of a subject eye is in development. For example, an ophthalmic device of US Patent No. 9072460 (hereinbelow "Patent Document 1") can acquire a tomographic image of an anterior segment and a tomographic image of a fundus of a subject eye. This ophthalmic device includes a light source for the anterior segment, and a light source for the fundus that uses a wavelength different from that of the light source for the anterior segment. Light from the light source for the anterior segment travels through an optical path for the anterior segment where a scanner and an objective lens are arranged, and is irradiated to the anterior segment of the subject eye. Light from the light source for the fundus travels through the scanner and branches from the optical path for the anterior segment to an optical path for the fundus where a relay lens is arranged, merges again into the optical path for the anterior segment from the optical path for the fundus, and is irradiated to the fundus of the subject eye through the same objective lens as aforementioned. That is, a part of the optical path for the fundus is shared with the optical path for the anterior segment. This ophthalmic device acquires both the anterior segment tomographic image and the fundus tomographic image by sharing the scanner and the objective lens for the anterior segment and the fundus and on the other hand providing the optical path for the fundus that branches from and merges into the optical path for the anterior segment between the scanner and the objective lens.

Further, an ophthalmic device of Japanese Patent Application Publication No. 2018-525046 (hereinbelow "Patent Document 2") also can acquire a tomographic image of the anterior segment and a tomographic image of the fundus of the subject eye. In this ophthalmic device, light from a light source for the anterior segment travels through a scanner and an objective lens for the anterior segment, and is irradiated to the anterior segment of the subject eye. Light from a light source for the fundus travels through a scanner for the fundus, penetrates through a relay lens, and is irradiated to the fundus of the subject eye through the same objective lens as aforementioned. That is, the ophthalmic device of Patent Document 2 acquires both the anterior segment tomographic image and the fundus tomographic image by respectively providing the scanner for the anterior segment and the scanner for the fundus while on the other hand sharing the objective lens.

### SUMMARY

In the conventional ophthalmic devices as described above, the light from multiple light sources is irradiated to different measurement ranges of the subject eye to acquire tomographic information for each of the different measurement ranges of the subject eye. Due to this, there is a problem that a configuration for acquiring the tomographic information for one of the measurement ranges adversely affects another of the measurement ranges upon acquiring tomographic information thereof, and the acquired tomographic information is contaminated by noise. The description herein provides an art configured to suppress noise contamination of tomographic information in an ophthalmic device capable of acquiring the tomographic information for each of different measurement ranges of a subject eye.

In one aspect of the present disclosure, an ophthalmic device may comprise: a first light source configured to output first light; a second light source configured to output second light; a first scanning optical system configured to scan the first light outputted from the first light source in a first range of a subject eye; a second scanning optical system configured to scan the second light outputted from the second light source in a second range of the subject eye that is different from the first range; a first interferometer configured to acquire tomographic information of the first range based on first interference light obtained from reflected light of the first light reflected on the subject eye; and a second interferometer configured to acquire tomographic information of the second range based on second interference light obtained from reflected light of the second light reflected on the subject eye. A central wavelength of the first light and a central wavelength of the second light are different. The first scanning optical system comprises a first objective lens unit configured to irradiate the first light to the first range of the subject eye. The second scanning optical system comprises a second objective lens unit configured to irradiate the second light to the second range of the subject eye. The first light does not penetrate the second objective lens unit but penetrates the first objective lens unit, and the second light does not penetrate the first objective lens unit but penetrates the second objective lens unit.

The above ophthalmic device has the first objective lens unit dedicated to acquiring the tomographic information of the first range arranged, and the second objective lens unit dedicated to acquiring the tomographic information of the second range also arranged. Due to this, noise contamination in the tomographic information can be suppressed as compared to the conventional ophthalmic devices.

That is, in the conventional ophthalmic devices, the objective lens is shared by anterior segment measurement and fundus measurement, and both the light of the light source for the anterior segment and the light of the light source for the fundus are irradiated to the subject eye through the same objective lens. Since the devices have such a configuration, the light from the light source for the fundus is irradiated to the fundus of the subject eye through two lenses, namely the relay lens and the objective lens. As such, lens power (refractivity) required for scanning light in the fundus is dispersed by the two lenses. Due to this, a principal beam of the light between the relay lens and the objective lens becomes close to being parallel to an optical axis at all times irrelevant to a scan angle, thus enters the objective lens at an angle close to a right angle. As a result, a problem that noise generated by light reflected on a surface of the objective lens is undesirably captured in a tomographic image of the fundus occurs.

The above ophthalmic device has the first objective lens unit dedicated to acquiring the tomographic information of the first range and also the second objective lens unit dedicated to acquiring the tomographic information of the second range. Thus, the lens power for irradiating the light to the first range can be ensured by the first objective lens unit, and the lens power for irradiating the light to the second range can be ensured by the second objective lens unit. Due to this, an incident angle of the light to the first objective lens unit can be set relatively freely, and further, an incident angle of the light to the second objective lens unit can also be set relatively freely. Due to this, noise caused by the light reflected on surfaces of the objective lens units can be suppressed from contaminating the tomographic information.

The "objective lens units" as aforementioned may each be constituted of one lens (single lens) or a combination of multiple lenses that can be housed in a same lens barrel. In a case of configuring one of or both of the objective lens units by the combination of multiple lenses, the same may be configured by paired lenses capable of minimizing a number of interfaces with air, such as doublets and triplets.

In another aspect of the present disclosure, an ophthalmic device may comprise: a first light source configured to output first light; a second light source configured to output second light; a first scanning optical system configured to scan the first light outputted from the first light source in a first range of a subject eye; a second scanning optical system configured to scan the second light outputted from the second light source in a second range of the subject eye that is different from the first range; a first interferometer configured to acquire tomographic information of the first range based on first interference light obtained from reflected light of the first light reflected on the subject eye; a second interferometer configured to acquire tomographic information of the second range based on second interference light obtained from reflected light of the second light reflected on the subject eye; a measuring window configured to face the subject eye; and a first dichroic mirror configured to reflect the first light but allow the second light to penetrate. The first range is an anterior segment of the subject eye. The second range is a fundus of the subject eye. A central wavelength of the first light and a central wavelength of the second light are different. A first optical path being an optical path of the first light includes an overlapped section that overlaps with a second optical path being an optical path of the second light and a first nonoverlapped section that does not overlap with the second optical path. The second optical path includes the overlapped section and a second nonoverlapped section that does not overlap with the first optical path. The overlapped section includes a first overlapped section that includes a section connecting the subject eye and the measuring window. The first dichroic mirror is arranged at a first position where the first overlapped section branches into the first nonoverlapped section and the second nonoverlapped section, and a back surface of the first dichroic mirror comprises Anti-Reflection (AR) coating for a wavelength bandwidth of the first light.

In the above ophthalmic device, the first light outputted from the first light source for the anterior segment is reflected on the first dichroic mirror, travels through the measuring window, and irradiated to the anterior segment of the subject eye. Due to this, occurrence of ghost noise caused by irradiating light having penetrated the dichroic mirror to the anterior segment can be avoided. That is, when light penetrated through the first dichroic mirror is irradiated to the anterior segment of the subject eye, light reflected on the back surface of the first dichroic mirror would also be irradiated to the anterior segment of the subject eye. Since light reflecting strength of an iris is strong, the light reflected on the back surface of the first dichroic mirror would be reflected at a high rate by the iris, by which the ghost noise contaminates the tomographic information. In the above ophthalmic device, since the first light reflected on the first dichroic mirror is irradiated to the anterior segment of the subject eye when the tomographic information of the anterior segment of the subject eye is to be acquired, contamination of the tomographic information by the ghost noise can be suppressed.

Further, in the above ophthalmic device, the back surface of the first dichroic mirror comprises the AR coating for the wavelength bandwidth of the first light. Due to this, when the tomographic information of the anterior segment of the subject eye is to be acquired, intensity of reflected light of the first light reflected on the back surface of the first dichroic mirror is thereby suppressed, and noise contamination of the tomographic information can further be suppressed.

### BRIEF DESCRIPTION OF DRAWINGS

FIG. 1 is a schematic configurational diagram of optical systems of an ophthalmic device of a first embodiment;
FIG. 2 is a diagram for explaining an anterior segment scanning optical system of the ophthalmic device of the first embodiment;
FIG. 3 is a diagram for explaining a fundus scanning optical system of the ophthalmic device of the first embodiment;
FIG. 4 is a diagram for explaining a light receiving system for receiving light reflected from a subject eye in a total refractivity measuring optical system of the ophthalmic device of the first embodiment;
FIG. 5 is a diagram for explaining a front monitoring optical system of the ophthalmic device of the first embodiment;
FIG. 6 is a diagram for explaining a position detection floodlighting system of the ophthalmic device of the first embodiment;
FIG. 7 is a diagram for explaining a position detection light receiving system of the ophthalmic device of the first embodiment;
FIG. 8 is a diagram for explaining a vision fixing target optical system of the ophthalmic device of the first embodiment;
FIG. 9 is an example of a tomographic image in which reflection noise of an objective lens is undesirably captured in an ophthalmic device of conventional techniques;
FIG. 10 is an example of an image in which reflection noise of the objective lens is undesirably captured upon total refractivity measurement in the ophthalmic device of the conventional techniques;
FIG. 11 is a diagram for explaining back surface reflection of a dichroic mirror;
FIG. 12 is an example of a tomographic image of an anterior segment of a subject eye E in which ghost noise caused by the back surface reflection of the dichroic mirror is undesirably captured;
FIG. 13 is a diagram for explaining back surface reflection of light having penetrated a front surface of the dichroic mirror; and
FIG. 14 is examples of tomographic images of the anterior segment acquired with different iris signal intensity levels.

### DETAILED DESCRIPTION

In an embodiment of the ophthalmic device disclosed herein, a first optical path being an optical path of the first light may include an overlapped section that overlaps with a second optical path being an optical path of the second light, and a first nonoverlapped section that does not overlap with the second optical path, the second optical path may include the overlapped section and a second nonoverlapped section that does not overlap with the first optical path, the first objective lens unit may be arranged in the first nonoverlapped section, and the second objective lens unit may be arranged in the second nonoverlapped section. According to such a configuration, the first light can be irradiated to the subject eye through the first objective lens unit, and the second light can be irradiated to the subject eye through the second objective lens unit.

In an embodiment of the ophthalmic device disclosed herein, the first scanning optical system may comprise a first scanner configured to scan the first light outputted from the first light source, the second scanning optical system may comprise a second scanner configured to scan the second light outputted from the second light source, the first scanner may be arranged in the first nonoverlapped section, and the second scanner may be arranged in the second nonoverlapped section. According to such a configuration, the first scanner can be optimized for scanning light in the first range, and further the second scanner can be optimized for scanning light in the second range.

In an embodiment of the ophthalmic device disclosed herein, the ophthalmic device may further comprise a scanner configured to scan the first light outputted from the first light source and configured to scan the second light outputted from the second light source, wherein the scanner may be arranged in the overlapped section and may be shared by the first scanning optical system and the second scanning optical system, the first objective lens unit may be arranged between the scanner and the subject eye, and the second objective lens unit may be arranged between the scanner and the subject eye. According to such a configuration, since the scanner is shared by the first and second scanning optical systems, a number of components of the optical systems can be reduced. Further, the ophthalmic device itself can be made more compact by configuring the scanner as a shared component.

In an embodiment of the ophthalmic device disclosed herein, the ophthalmic device may further comprise: a measuring window configured to face the subject eye; and a first dichroic mirror configured to reflect the first light but allow the second light to penetrate. The first range may be an anterior segment of the subject eye. The second range may be a fundus of the subject eye. The overlapped section may include a first overlapped section that includes a section connecting the subject eye and the measuring window. The first dichroic mirror may be arranged at a first position where the first overlapped section branches into the first nonoverlapped section and the second nonoverlapped section. According to such a configuration, when tomographic information of the anterior segment of the subject eye is to be acquired, the first light having been reflected on the first dichroic mirror is irradiated to the anterior segment of the subject eye. Due to this, occurrence of ghost noise caused by irradiating light having penetrated the dichroic mirror to the anterior segment can be avoided.

In an embodiment of the ophthalmic device disclosed herein, a back surface of the first dichroic mirror may comprise Anti-Reflection (AR) coating for a wavelength bandwidth of the first light. According to such a configuration, when the tomographic information of the anterior segment of the subject eye is to be acquired, intensity of reflected light of the first light reflected on the back surface of the first dichroic mirror is suppressed, and noise contamination of the tomographic information can further be suppressed.

In an embodiment of the ophthalmic device disclosed herein, the ophthalmic device may further comprise a second dichroic mirror configured to reflect the first light but allow the second light to penetrate. The overlapped section may include a second overlapped section that includes a section connecting the scanner and a second position where the first nonoverlapped section and the second nonoverlapped section merge and branch, and the second dichroic mirror may be arranged at the second position. According to such a configuration, when the tomographic information of the anterior segment of the subject eye is to be acquired, the first light reflected on the second dichroic mirror is irradiated to the anterior segment of the subject eye. Due to this, the occurrence of the ghost noise caused by irradiating the light having penetrated the dichroic mirror to the anterior segment can be avoided.

In an embodiment of the ophthalmic device disclosed herein, a back surface of the second dichroic mirror may comprise Anti-Reflection (AR) coating for a wavelength bandwidth of the first light. According to such a configuration, when the tomographic information of the anterior segment of the subject eye is to be acquired, intensity of reflected light of the first light reflected on the back surface of the second dichroic mirror is suppressed, and the noise contamination of the tomographic information can further be suppressed.

In an embodiment of the ophthalmic device disclosed herein, the ophthalmic device may further comprise a third dichroic mirror arranged at a third position, which is a position at one end of the second overlapped section and different from the second position, wherein the third dichroic mirror may be configured to reflect the first light but allow the second light to penetrate. Further, a back surface of the third dichroic mirror may comprise Anti-Reflection (AR) coating for a wavelength bandwidth of the first light. According to such a configuration, when the tomographic information of the anterior segment of the subject eye is to be acquired, intensity of reflected light of the first light reflected on the back surface of the third dichroic mirror is suppressed, and the noise contamination of the tomographic information can be suppressed.

### EMBODIMENT

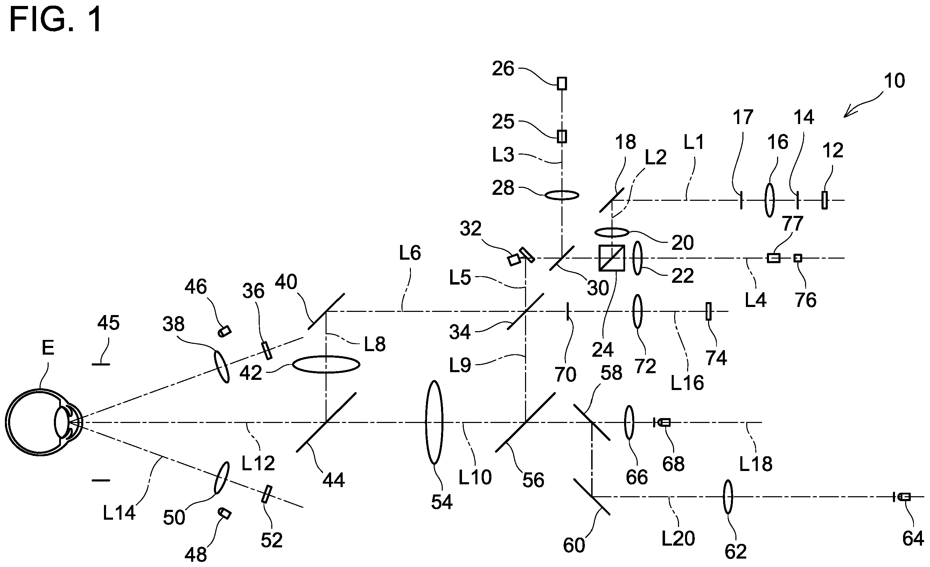

Hereinbelow, an ophthalmic device of an embodiment will be described. The ophthalmic device of the present embodiment is configured to acquire a tomographic image of an anterior segment of a subject eye E, acquire a tomographic image of a fundus of the subject eye E, and measure total refractivity of the subject eye E. Due to this, information for comprehensive diagnosis of a condition of the subject eye E can be acquired by one ophthalmic device. Due to being configured to perform the aforementioned multiple functions, the ophthalmic device of the present embodiment includes an optical system 10 shown in FIG. 1. That is, the optical system 10 includes an anterior segment scanning optical system, a fundus scanning optical system, a total refractivity measuring optical system, a front monitoring optical system, a position detection light floodlighting optical system, a position detection light receiving optical system, and a vision fixing target optical system. Hereinbelow, each optical system will be described.

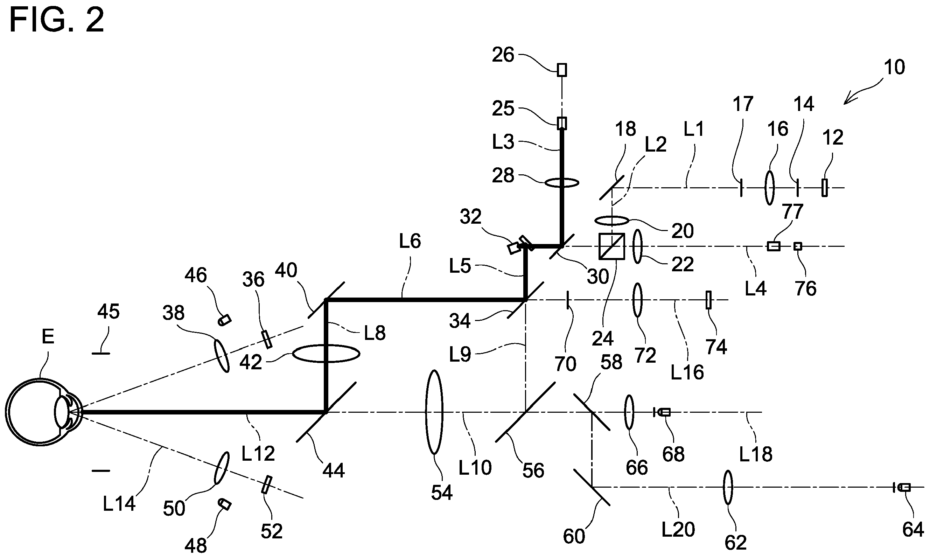

As shown in FIG. 2, the anterior segment scanning optical system is constituted of an anterior segment light source 26 (being an example of first light source), a collimate lens 28, a dichroic mirror 30, a 2D scanner 32, a dichroic mirror 34, a total reflection mirror 40, an objective lens 42 (being an example of first objective lens unit), and a dichroic mirror 44.

The anterior segment light source 26 is a wavelength sweeping light source, and a wavelength (wavenumber) of light outputted therefrom changes at a predetermined cycle. The anterior segment light source 26 is capable of outputting light with a long wavelength, thus is capable of outputting light with a central wavelength of 0.95µm or more and 1.80µm or less, for example. In the present embodiment, the anterior segment light source 26 outputs light with the central wavelength of 1.31µm. When the light with the long wavelength is used, the light can more easily penetrate through highly diffusive tissues such as clouding in a crystalline lens, a ciliary body, a conjunctiva, and a sclera, and further, due to difficulty of the light reaching a fundus because of a large water absorption, stronger light can be irradiated. Due to this, by outputting the light with the central wavelength of 0.95µm or more from the anterior segment light source 26, percentage of the light reaching tissues constituted of diffusive substances can be increased. Further, since the light with the central wavelength of 0.95µm or more and 1.80µm or less is less decentralized by water, an anterior segment OCT image with good image quality can be acquired by irradiating light in this range to the subject eye E. Further, by outputting the light with the central wavelength of 1.80µm or less from the anterior segment light source 12, a target site can be measured with high sensitivity using indium-gallium-arsenide (InGaAs)-based light receiving elements. In the present embodiment, by outputting the light with the central wavelength of 0.95µm or more and 1.80µm or less from the anterior segment light source 26, the tomographic image of the anterior segment of the subject eye E can suitably be captured.

The light outputted from the anterior segment light source 26 (being an example of first light) travels through optical fiber that is not shown and an interferometer 25, and enters the collimate lens 28. The collimate lens 28 is configured to transform the light outputted from the anterior segment light source 26 into parallel light. The light that has been transformed into the parallel light in the collimate lens 28 is reflected on the dichroic mirror 30 and enters the 2D scanner 32. The 2D scanner 32 is configured to scan inputted light in two directions, namely x and y directions, relative to the anterior segment of the subject eye E. In the present embodiment, a Galvano scanner is used as the 2D scanner 32. Scanners other than the Galvano scanner may be used as the 2D scanner 32, and for example, a MEMS mirror configured to perform biaxial scan may be used. The light outputted from the 2D scanner 32 travels on the dichroic mirror 34 and the total reflection mirror 40, and enters the objective lens 42. The light that entered the objective lens 42 penetrates the objective lens 42, is reflected on the dichroic mirror 44, condenses in a vicinity of the anterior segment of the subject eye E, and is irradiated to the subject eye E. In the present embodiment, the 2D scanner 32 is arranged at a rear focal point of the objective lens 42. Due to this, the light outputted from the 2D scanner 32 reaches the subject eye E parallel to an optical axis (optical path L12). That is, in the present embodiment, the light is scanned by telecentric scan on the anterior segment of the subject eye E. A measuring window 45 (an opening of eyepiece) is arranged between the dichroic mirror 44 and the subject eye E. The measuring window 45 is arranged at a position that is to face the subject eye E upon measurement. The measuring window 45 is provided in a housing that is not shown, and the optical system 10 is housed in this housing.

The light reflected on the anterior segment of the subject eye E travels through a same path as the aforementioned path, and is guided to the interferometer 25 (being an example of first interferometer) through the optical fiber that is not shown. The interferometer 25 is configured to output an interference signal by multiplexing the light reflected on the anterior segment of the subject eye E and reference light that is generated separately from the light outputted from the anterior segment light source 26 and detecting interference light obtained by this multiplexing. In the ophthalmic device of the present embodiment, the tomographic image of the anterior segment of the subject eye E is acquired by processing the interference signal outputted from the interferometer 25.

As it is apparent from the foregoing description, in the anterior segment scanning optical system, an optical path L3, a part of an optical path L4 (more specifically, in a range between the dichroic mirror 30 and the 2D scanner 32), an optical path L5, an optical path L6, an optical path L8 (that is, a range between the total reflection mirror 40 and the dichroic mirror 44), and the optical path L12 constitute the path through which the light travels.

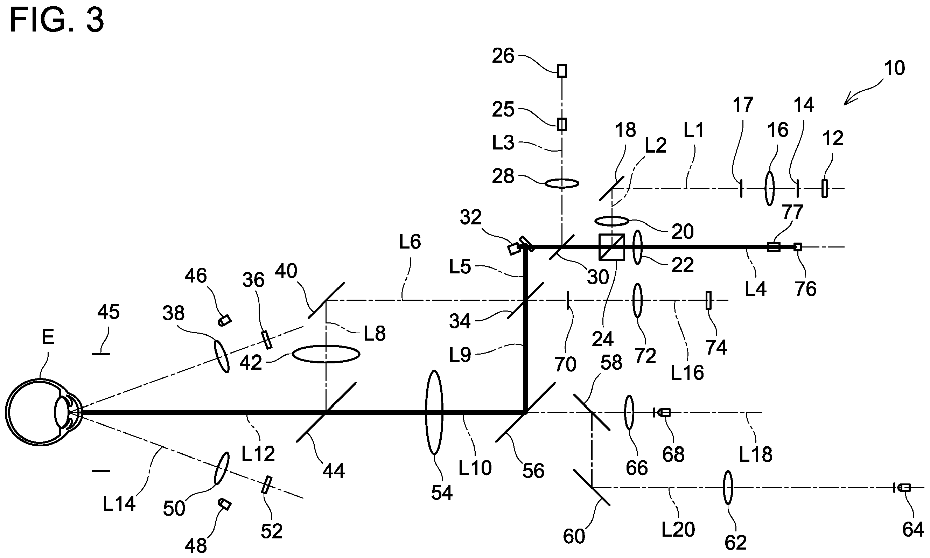

As shown in FIG. 3, the fundus scanning optical system is constituted of a fundus light source 76 (being an example of second light source), a lens 22, a polarization beam splitter 24, the dichroic mirror 30, the 2D scanner 32, the dichroic mirror 34, a dichroic mirror 56, an objective lens 54 (being an example of second objective lens unit), and the dichroic mirror 44.

The fundus light source 76 is a fixed wavelength light source. The fundus light source 76 is configured to output light having the central wavelength different from that of the light outputted from the anterior segment light source 26, and can for example output light with the central wavelength of 0.40µm or more and 1.15µm or less. Further, for example, the fundus light source 76 may output light having its half value width in a wavelength range different from a wavelength range of a half value width of the light which the anterior segment light source 12 outputs. In the present embodiment, the fundus light source 76 outputs the light with the central wavelength of 0.83µm. The light with the central wavelength of 0.40µm or more and 1.15µm or less has a high intraocular penetration rate. Due to this, by outputting the light with the central wavelength of 0.40µm or more and 1.15µm or less from a light source, this light can sufficiently be irradiated to the fundus of the subject eye E. Further, the light with the central wavelength of 0.40µm or more and 0.95µm or less has high sensitivity with silicon-based light receiving elements. Further, the light with the central wavelength of 0.95µm or more and 1.15µm or less is less decentralized in its wavelength by water, a fundus OCT image with good image quality can be acquired by irradiating light in this range to the subject eye E. Thus, by outputting the light with the central wavelength of 0.40µm or more and 1.15µm or less from the light source 76, the tomographic image of the fundus of the subject eye E can suitably be captured.

The light outputted from the fundus light source 76 (being an example of second light) is outputted through an optical fiber that is not shown via an interferometer 77 after having been adjusted to light having only a P-polarization light component. The light outputted from the optical fiber penetrates through the lens 22, the polarization beam splitter 24, and the dichroic mirror 30 and enters the 2D scanner 32. The 2D scanner 32 scans the inputted light in two directions, namely x and y directions, relative to the fundus of the subject eye E. The light outputted from the 2D scanner 32 penetrates through the dichroic mirror 34, is reflected on the dichroic mirror 56, and enters the objective lens 54. The light inputted to the objective lens 54 penetrates through the objective lens 54 then through the dichroic mirror 44, condenses in a vicinity of the fundus of the subject eye E, and is irradiated to the fundus of the subject eye E. In the ophthalmic device of the present embodiment, power of the objective lens 54 is set such that convergent light is irradiated to the fundus of the subject eye E. Further, a position of an output end of the optical fiber from which the light outputted from the fundus light source 76 exits is configured movable in an optical axis direction (direction in which the optical path L4 extends; that is, an optical path length thereof is configured adjustable), thus it is configured to move in accordance with refractivity of the subject eye E.

The light reflected on the fundus of the subject eye E travels through a same path as the aforementioned path, and is guided to the interferometer 77 (being an example of second interferometer) through the optical fiber that is not shown. The interferometer 77 is configured to output an interference signal by multiplexing the light reflected on the fundus of the subject eye E and reference light that is generated separately from the light outputted from the fundus light source 76 and detecting interference light obtained by this multiplexing. In the ophthalmic device of the present embodiment, the tomographic image of the fundus of the subject eye E is acquired by processing the interference signal outputted from the interferometer 77.

As it is apparent from the foregoing description, in the fundus scanning optical system, the optical path L4, the optical path L5, an optical path L9, an optical path L10, and the optical path L12 constitute the path through which the light travels. As such, in the anterior segment scanning optical system and the fundus scanning optical system, a part of the optical path L4 (more specifically, in the range between the dichroic mirror 30 and the 2D scanner 32), the optical path L5, and a part of the optical path L12 are configured as an overlapped path (being an example of overlapped section), the optical path L8, the optical path L6, and the optical path L3 are configured as a path dedicated to the light in the anterior segment scanning optical system (being an example of first nonoverlapped section), and a remaining part of the optical path L4, the optical path L9, and the optical path L10 are configured as a path dedicated to the light in the fundus scanning optical system (being an example of second nonoverlapped section).

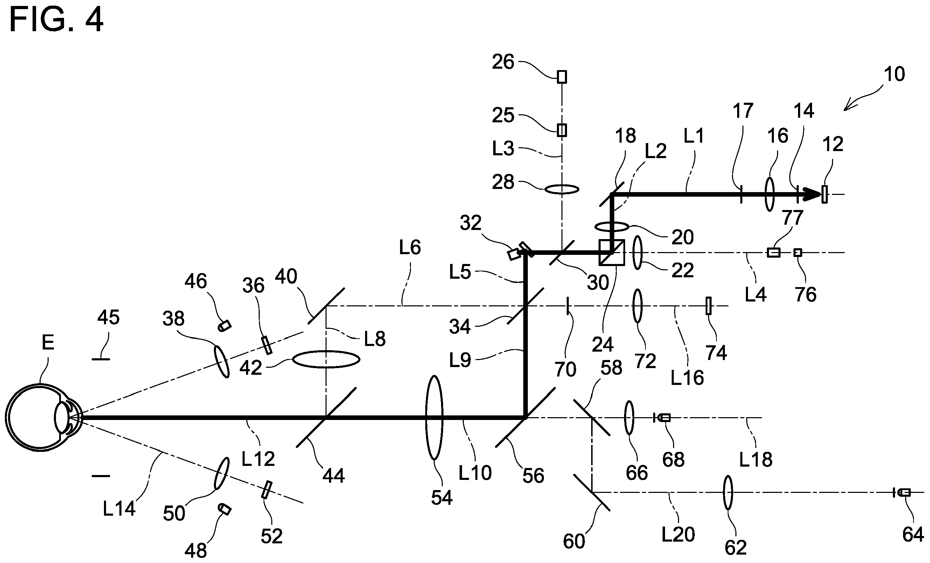

Next, the total refractivity measuring optical system will be described. A floodlighting system in the total refractivity measuring optical system that floods light to the subject eye E has a same configuration as a floodlighting system in the fundus scanning optical system. Due to this, a light receiving system in the total refractivity measuring optical system will be described. As shown in FIG. 4, the total refractivity measuring optical system includes the dichroic mirror 44, the objective lens 54, the dichroic mirror 56, the dichroic mirror 34, the 2D scanner 32, the dichroic mirror 30, the polarization beam splitter 24, a lens 20, a mirror 18, an aperture 17, a lens 16, a donut lens 14, and a 2D sensor 12.

As it is apparent from comparison of FIGS. 3 and 4, a path of light diffused by the fundus of the subject eye E is same as that of the fundus scanning optical system from the dichroic mirror 44 to the polarization beam splitter 24. At the polarization beam splitter 24, only a S-polarization light component of the light diffused by the fundus of the subject eye E is reflected and irradiated to the mirror 18 through the lens 20. The light irradiated to the mirror 18 penetrates through the aperture 17, the lens 16, and the donut lens 14, and forms a ring-shaped image on a light receiving surface of the 2D sensor 12. Total refractivity of the subject eye E is calculated based on this ring-shaped image formed in the 2D sensor 12. In the present embodiment, the light diffused by the fundus of the subject eye E forms the image in the ring shape on the light receiving surface of the 2D sensor 12 by using the donut lens 14, however, no limitation is made hereto, and a lens array may be used instead of the donut lens 14, and an image in a dot pattern may be formed on the light receiving surface of the 2D sensor 12. The aperture 17, the lens 16, the donut lens 14, and the 2D sensor 12 are integrated and configured movable in an optical axis direction (optical path L1), thus it is configured to move in accordance with the total refractivity of the subject eye E.

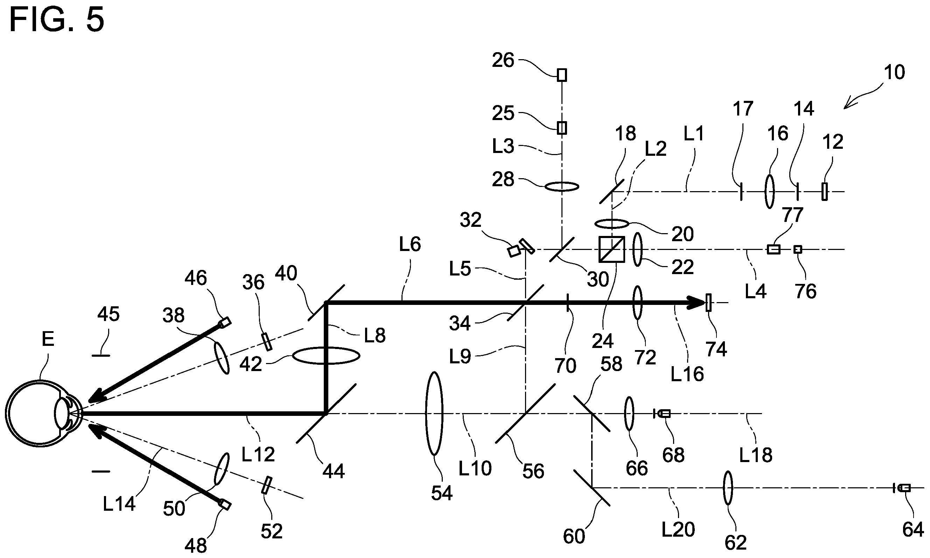

As shown in FIG. 5, the front monitoring optical system is constituted of LEDs 46, 48, the dichroic mirror 44, the objective lens 42, the total reflection mirror 40, the dichroic mirror 34, an aperture 70, a lens 72, and a 2D sensor 74.

The LEDs 46, 48 are arranged on obliquely front sides of the subject eye E and are configured to illuminate the anterior segment of the subject eye E. The LEDs 46, 48 are configured to irradiate light with the central wavelength of 0.76µm to the subject eye E. The light reflected on the subject eye E is reflected on the dichroic mirror 44, penetrates through the objective lens 42, is reflected on the total reflection mirror 40, penetrates the dichroic mirror 34, the aperture 70, and the lens 72, and forms a front image of the anterior segment on the 2D sensor 74. The image of the anterior segment of the subject eye E captured by the 2D sensor 74 is displayed on a display device that is not shown. The aperture 70 is arranged at the rear focal point of the objective lens 42, and is configured such that its image magnification does not change even when the anterior segment image is defocused.

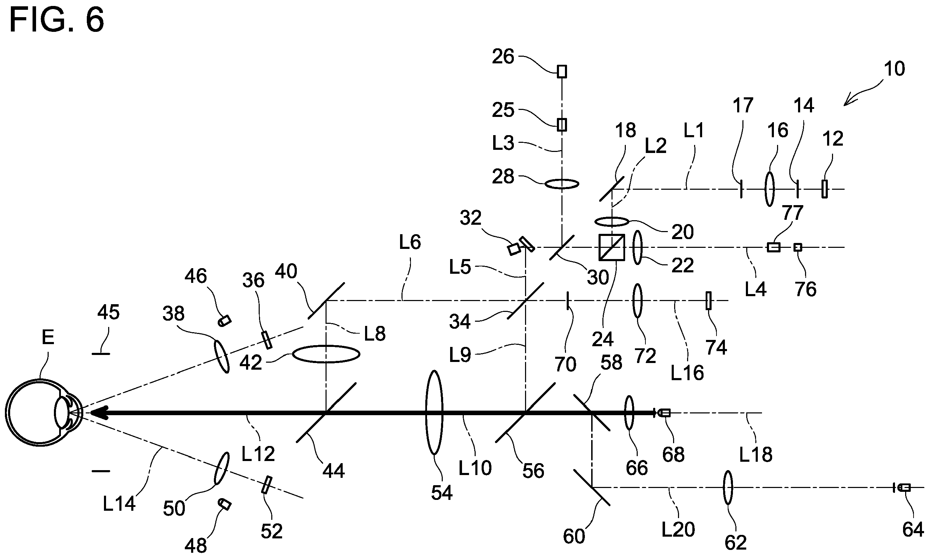

As shown in FIG. 6, the position detection light floodlighting optical system is constituted of a LED 68, a lens 66, a dichroic mirror 58, the dichroic mirror 56, the objective lens 54, and the dichroic mirror 44. The LED 68 is configured to output light with the central wavelength of 0.94µm The light outputted from the LED 68 penetrates through the lens 66, the dichroic mirrors 58, 56, the objective lens 54, and the dichroic mirror 44, and is irradiated to a cornea of the subject eye E. The light irradiated to the subject eye E is mirror-reflected on a surface of the cornea of the subject eye E, and a virtual image of a light emitting surface of the LED 68 is formed on an extended line of a corneal apex.

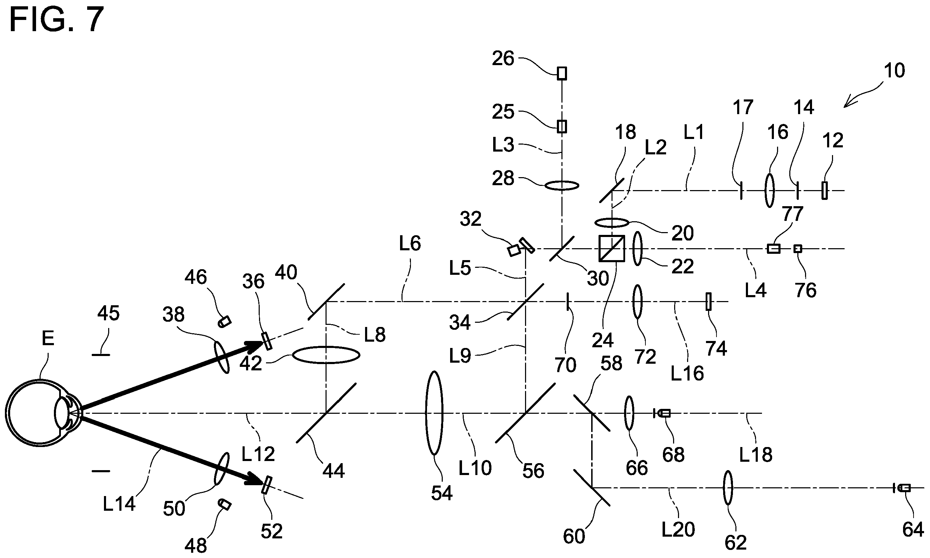

The position detection light receiving optical system is configured to detect a corneal apex position in a direction (i.e., lateral direction) perpendicularly intersecting the optical axis (optical path L12) and detect a corneal apex position in the optical axis direction (i.e., depth direction). The position detection light receiving optical system is constituted of a lens 50, a 2D sensor 52, a lens 38, and a 2D sensor 36 (see FIG. 7). The lens 50 and the 2D sensor 52 are arranged on one of the obliquely front sides of the subject eye E. The lens 38 and the 2D sensor 36 are also arranged on the other of the obliquely front sides of the subject eye E. The lens 38 and the 2D sensor 36 are arranged at positions symmetric to the lens 50 and the 2D sensor 52 with respect to the optical axis (optical path L12). Light reflected at a position that is slightly offset from the corneal apex of the subject eye E is reflected in an oblique direction, penetrates the lens 50, and a virtual image of the light emitting surface of the LED 68 is projected on the 2D sensor 52. Similarly, the light reflected at a position that is slightly offset from the corneal apex of the subject eye E penetrates the lens 38, and a virtual image of the light emitting surface of the LED 68 is projected on the 2D sensor 36. In the ophthalmic device of the present embodiment, the corneal apex position in the direction (i.e., lateral direction) perpendicularly intersecting the optical axis (optical path L12) and the corneal apex position in the optical axis direction (i.e., depth direction) are detected based on the virtual images of the light emitting surface of the LED 68 detected by the 2D sensors 36, 52.

After the position of the corneal apex of the subject eye E is detected based on detection results of the 2D sensor 36 and the 2D sensor 52, the housing that houses the optical system 10 is driven by a driver device that is not shown, and the housing is positioned at a measurement position relative to the corneal apex of the subject eye E. Due to this, the measuring window 45 is positioned relative to the subject eye E, and the objective lenses 42, 54 of the optical system 10 are thereby positioned. When the measuring window 45 (objective lenses 42, 54 of the optical system 10) is positioned relative to the subject eye E, positions of the measuring window 45 and the objective lenses 42, 54 relative to the subject eye E do not change while acquiring the tomographic images of the anterior segment and the fundus of the subject eye E and the total refractivity thereof.

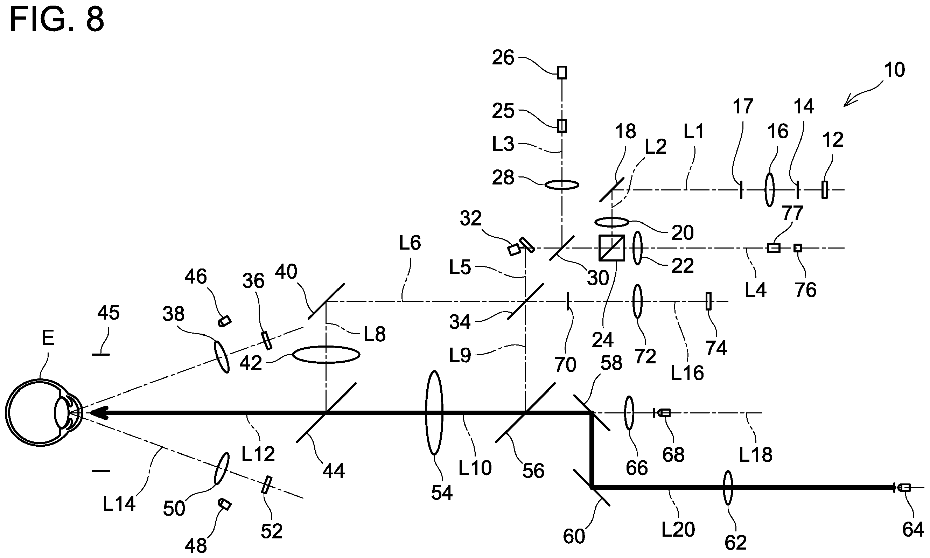

As shown in FIG. 8, the vision fixing target optical system is constituted of a LED 64, a lens 62, a mirror 60, the dichroic mirrors 58, 56, the objective lens 54, and the dichroic mirror 44. The LED 64 is configured to output white light. The light from the LED 64 penetrates an image film on which a symbol for fixing vision of an examined person is printed, and is reflected on the mirror 60. The light reflected on the mirror 60 is further reflected on the dichroic mirror 58, penetrates through the dichroic mirror 56, the objective lens 54, and the dichroic mirror 44, and is irradiated toward the subject eye E. The LED 64 and the image film are configured movable in an optical axis direction (i.e., direction along an optical path L20), thus positions of the LED 64 and the image film are configured to be adjusted in accordance with the total refractivity of the subject eye E.

As it is apparent from the foregoing description, in the ophthalmic device of the present embodiment, no objective lens is arranged on the optical path L12 between the subject eye E and the dichroic mirror 44, but rather, the objective lens 42 dedicated to the anterior segment scanning optical system is arranged on the optical path L8 dedicated to the anterior segment scanning optical system and further the objective lens 54 dedicated to the fundus scanning optical system is arranged on the optical path L10 dedicated to the fundus scanning optical system. Due to this, as compared to conventional techniques, noise generated by the reflected light that reflects on surfaces of the objective lenses 42, 54 can be suppressed from contaminating the tomographic images.

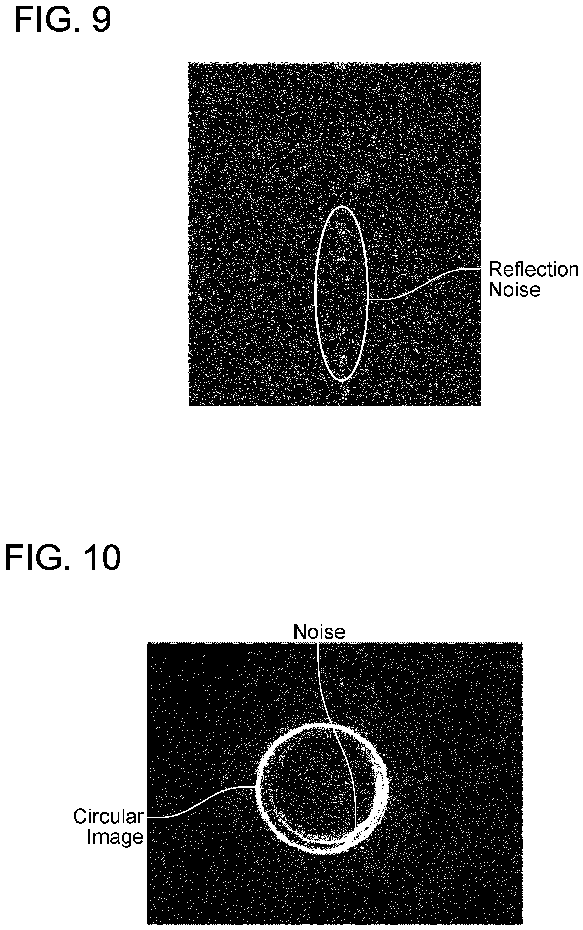

That is, if a configuration in which a common objective lens is arranged on the optical path L12 between the subject eye E and the dichroic mirror 44 and a relay lens is arranged on the optical path dedicated to the fundus scanning optical system as in the conventional techniques is employed, the fundus scanning optical system would result in having arranged therein two lenses, namely the objective lens and the relay lens. That is, lens power (refractivity) required for scanning light in the fundus is decentralized by these two lenses, namely the objective lens and the relay lens. Due to this, a principal beam of the light between the relay lens and the objective lens becomes close to being parallel to the optical axis at all times irrelevant to a scan angle, thus enters the objective lens arranged on a subject eye side at an angle close to a right angle. As a result, a problem that the noise generated by the light reflected on the surface of the objective lens (that is, on a surface opposite from the subject eye side) is undesirably captured in the tomographic image of the fundus occurs (see FIG. 9). However, in the fundus scanning optical system of the ophthalmic device of the present embodiment, the dedicated objective lens 54 is arranged on the optical path L10 while no lens is arranged between the objective lens 54 and the subject eye E, and further, no lens is arranged also between the 2D scanner 32 and the objective lens 54. Due to this, the lens power (refractivity) required for scanning light in the fundus can be ensured by the objective lens 54. As a result, an incident angle of the light to the objective lens 54 can be adjusted, and light reflected on the objective lens 54 can be suppressed from contaminating the tomographic image. Similarly in the anterior segment scanning optical system of the ophthalmic device of the present embodiment, the dedicated objective lens 42 is arranged on the optical path L8 while no lens is arranged between the objective lens 42 and the subject eye E, and further, no lens is arranged also between the 2D scanner 32 and the objective lens 42. Due to this, the lens power (refractivity) required for scanning light in the anterior segment can be ensured by the objective lens 42. As a result, an incident angle of the light to the objective lens 42 can be adjusted, and light reflected on the objective lens 42 can be suppressed from contaminating the tomographic image.

Further, if the configuration in which a common objective lens is arranged on the optical path L12 between the subject eye E and the dichroic mirror 44 and the relay lens is arranged on the optical path dedicated to the fundus scanning optical system as in the conventional techniques is employed, the light receiving system of the total refractivity measuring optical system results in having arranged therein two lenses, namely the objective lens and the relay lens. Due to this, the principal beam of the light between the relay lens and the objective lens becomes close to being parallel to the optical axis, thus enters the objective lens arranged on the subject eye side at an angle close to the right angle. As a result of this, not only an image reflected on the fundus of the subject eye E but also an image generated by light reflected on the surface of the objective lens (that is, on the surface opposite from the subject eye side) become contaminating noise. For example, as shown in FIG. 10, not only a circular image located on an outermost side (image generated by the light reflected on the fundus) but also a circular image on an inner side (image generated by the light reflected on the objective lens) are undesirably captured. In the light receiving system of the total refractivity measuring optical system in the ophthalmic device of the present embodiment, the objective lens 54 is arranged on the optical path L10, no lens is arranged between the objective lens 54 and the subject eye E, and further, no lens is arranged also between the 2D scanner 32 and the objective lens 54. Due to this, the light reflected on the surface of the objective lens 54 can be suppressed from contaminating images captured by the refractivity measuring optical system.

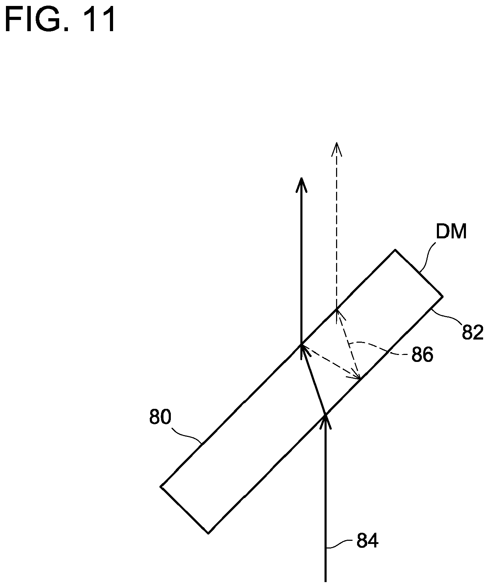

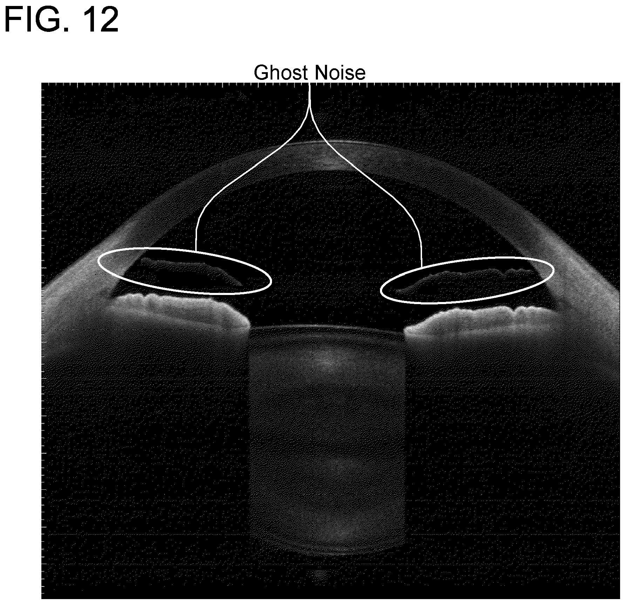

Further, in the ophthalmic device of the present embodiment, the dichroic mirrors 30, 34, 44, 56 are used to branch and merge the optical path of the anterior segment scanning optical system and the optical path of the fundus scanning optical system. Here, each of the dichroic mirror 30 (being an example of third dichroic mirror as recited in the claims), the dichroic mirror 34 (being an example of second dichroic mirror as recited in the claims), and the dichroic mirror 44 (being an example of first dichroic mirror as recited in the claims) arranged on the optical path of the anterior segment scanning optical system is configured to reflect light of the anterior segment scanning optical system. Due to this, occurrence of ghost noise caused by an iris of the subject eye E in the anterior segment tomographic image can be suppressed. That is, as shown in FIG. 11, if light 84 of the anterior segment scanning optical system is configured to penetrate a dichroic mirror DM, light reflected on an output surface 80 side of the dichroic mirror DM is further reflected on an input surface 82 side of the dichroic mirror DM (i.e., back surface reflection), and this light 86 would be irradiated to the subject eye E. The iris of the subject eye E has strong light reflecting strength. Due to this, as shown in FIG. 12, not only an anterior segment image generated by the light 84 but also an image (ghost noise) generated by the light 86 being reflected by the iris would be generated. In the ophthalmic device of the present embodiment, since the dichroic mirrors 30, 34, 44 arranged on the optical path of the anterior segment scanning optical system are configured to entirely reflect the light in the anterior segment scanning optical system, thus occurrence of the aforementioned ghost noise in the anterior segment tomographic image can be suppressed.

Back surfaces of the dichroic mirrors 30, 34, 44 may each have AR coating for the light of the anterior segment scanning optical system (such as the light with the central wavelength of 1.31µm). Due to this, reflecting strength of the light of the anterior segment scanning optical system reflected on the back surfaces of the dichroic mirrors 30, 34, 44 can be reduced, and the occurrence of the ghost noise in the anterior segment tomographic image can suitably be suppressed.

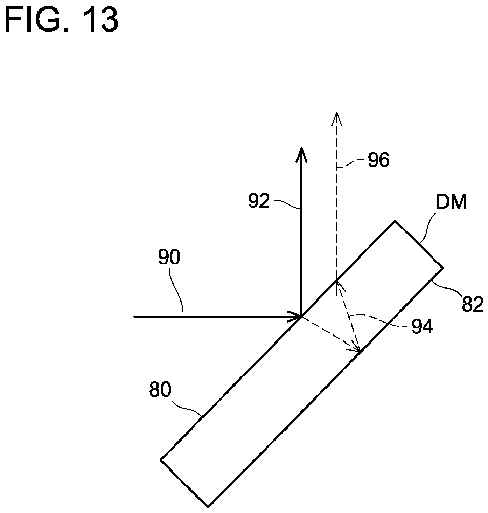

That is, even if the configuration is employed in which the light of the anterior segment scanning optical system is reflected on the dichroic mirror, a majority of light 90 having entered the front surface 80 of the dichroic mirror DM (such as 98%) is transformed into reflected light 92 as shown in FIG. 13, however, a part thereof (such as 2%) still penetrates through the dichroic mirror DM and is reflected on the back surface 82 of the dichroic mirror DM. Light 94 reflected on the back surface 82 of the dichroic mirror DM penetrates the front surface 80 of the dichroic mirror DM and light 96 having penetrated this front surface would be irradiated to the anterior segment of the subject eye E. Due to this, if intensity of the light 94 reflected on the back surface 82 of the dichroic mirror DM is strong, ghost noise generated by this reflected light 94 would occur in the anterior segment tomographic image. As such, the occurrence of the ghost noise in the anterior segment tomographic image can suitably be suppressed by providing the AR coating (of 1% or less, for example) for the light of the anterior segment scanning optical system on the back surfaces of the dichroic mirrors 30, 34, 44.

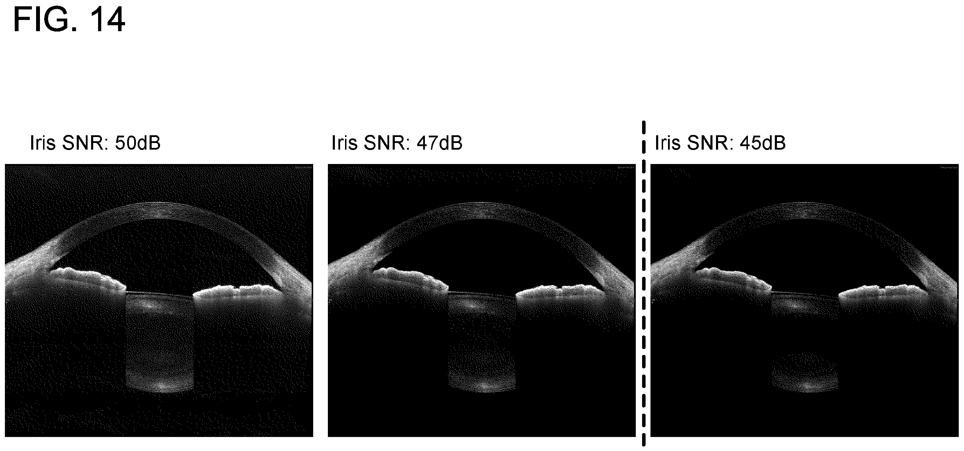

For example, the anterior segment has a greater variety of diffusive property in its tissues as compared to the fundus, and in order to capture transparent tissues such as the cornea or crystalline lens with sufficient contrast, signal intensity of iris diffusion is suitably at least 47dB or more in SN ratio as shown in FIG. 14, and 50dB or more is more suitable for capturing a range from the crystalline lens to the cornea in a single image. That is, a back surface of the cornea cannot clearly be recognized in an image with the signal intensity of iris diffusion of 45dB in SN ratio, however, the back surface of the cornea can be recognized in an image with the signal intensity of iris diffusion of 47dB in SN ratio, and further, the back surface of the cornea can be recognized clearly in an image with the signal intensity of iris diffusion of 50dB in SN ratio. On the other hand, if the AR coating is not given to the back surface of the dichroic mirror DM, the intensity of the reflected light 94 generated by the back surface reflection of the light having penetrated the dichroic mirror DM cannot be reduced sufficiently, and the ghost noise caused by the iris would occur in the anterior segment tomographic image. For example, even if a configuration is employed in which front surface reflectivity of the dichroic mirror DM is set to 97% and scanning light for the anterior segment is reflected with priority, back surface reflectivity of the dichroic mirror DM would be 4% if no AR coating is given to the back surface of the dichroic mirror DM. Due to this, the reflected light of the back surface of the dichroic mirror DM comes to have an extinction ratio of -44dB relative to reflected light of the front surface of the dichroic mirror DM, as a result of which the ghost noise would occur. On the other hand, if the AR coating is given to the back surface of the dichroic mirror DM, the back surface reflectivity of the dichroic mirror DM becomes 2% or less. Due to this, the reflected light of the back surface of the dichroic mirror DM comes to have the extinction ratio of -47dB relative to reflected light of the front surface of the dichroic mirror DM, as a result of which the ghost noise can be suppressed. When the light is to penetrate through the dichroic mirror DM (as in a case shown in FIG. 11), suppression of the ghost noise becomes even more difficult. For example, even if front surface penetration rate of the dichroic mirror DM is set to 97% and the AR coating is given to the back surface of the dichroic mirror DM (thus setting the back surface reflectivity to 0.5%), the extinction ratio of a ghost signal caused by the back surface reflection in the dichroic mirror DM would merely be -38dB, and the ghost noise cannot be suppressed with this ratio.

In view of the above, by providing the AR coating for a wavelength bandwidth of the light of the anterior segment scanning optical system (2% or less) on the back surface of the dichroic mirror DM, the extinction ratio of the ghost signal caused by the back surface reflection in the dichroic mirror DM can be brought to -47dB or less. That is, power of the light 96 reflected on the back surface 82 of the dichroic mirror DM becomes -47dB or less relative to power of the light 92 reflected on the front surface 80 of the dichroic mirror DM. Due to this, the occurrence of the ghost noise caused by the iris in the anterior segment tomographic image can be suppressed.

Here, AR coating is given to the back surface of the dichroic mirror DM generally for a wavelength of light that penetrates therethrough. That is, in a case of having the configuration of the optical system of the present embodiment, the back surface of the dichroic mirror DM would normally be given AR coating for the light of the fundus scanning optical system. A reason therefor is because an optical component of light that penetrates the mirror surface and is reflected on the back surface in light with a wavelength on a reflecting side (that is, the light of the anterior segment scanning optical system) is less likely to generate noise due to a majority thereof being reflected upon re-penetrating the mirror surface, thereby being significantly attenuated, however, an optical component that is reflected on the mirror surface and further reflected on the back surface in light with a wavelength on a penetrating side (that is, the light of the fundus scanning optical system) tends to become noise due to hardly attenuating at all upon re-penetrating the mirror surface after having been reflected on the back surface.

The iris signal intensity of the anterior segment tomographic image is 47dB or more in SN ratio, whereas signal intensity of a retinal pigment epithelium is about 30dB in the fundus tomographic image. Due to this, a problem of ghost noise of the retinal pigment epithelium does not occur in the fundus tomographic image even if the AR coating with priority to the light penetrating the back surface of the dichroic mirror DM (that is, the light of the fundus scanning optical system) is not given.

Further, in the aforementioned embodiment, the 2D scanner 32 is shared by the anterior segment scanning optical system and the fundus scanning optical system, however, no limitation is made to such an example. For example, a dedicated 2D scanner may be provided in the anterior segment scanning optical system, and another dedicated 2D scanner may be provided in the fundus scanning optical system. Further, in the aforementioned embodiment, one objective lens (single lens) 42 is arranged in the anterior segment scanning optical system, however, the objective lens to be arranged in the anterior segment scanning optical system may be constituted of a combination of multiple lenses (such as a pair of convex lens and concave lens, or aspheric lenses) housed in a same lens barrel. Similarly, the single objective lens 54 arranged in the fundus scanning optical system may be replaced with an objective lens constituted of a combination of multiple lenses housed in a same lens barrel. In such cases of configuring the objective lens unit by the combination of multiple lenses, those lenses may be joined together so that a number of interfaces with air is minimized.

While specific examples of the present disclosure have been described above in detail, these examples are merely illustrative and place no limitation on the scope of the patent claims. The technology described in the patent claims also encompasses various changes and modifications to the specific examples described above. The technical elements explained in the present description or drawings provide technical utility either independently or through various combinations. The present disclosure is not limited to the combinations described at the time the claims are filed.

## Claims

1. An ophthalmic device comprising:
a first light source configured to output first light;
a second light source configured to output second light;
a first scanning optical system configured to scan the first light outputted from the first light source in a first range of a subject eye;
a second scanning optical system configured to scan the second light outputted from the second light source in a second range of the subject eye that is different from the first range;
a first interferometer configured to acquire tomographic information of the first range based on first interference light obtained from reflected light of the first light reflected on the subject eye; and
a second interferometer configured to acquire tomographic information of the second range based on second interference light obtained from reflected light of the second light reflected on the subject eye,
wherein
a central wavelength of the first light and a central wavelength of the second light are different,
the first scanning optical system comprises a first objective lens unit configured to irradiate the first light to the first range of the subject eye,
the second scanning optical system comprises a second objective lens unit configured to irradiate the second light to the second range of the subject eye,
the first light does not penetrate the second objective lens unit but penetrates the first objective lens unit, and
the second light does not penetrate the first objective lens unit but penetrates the second objective lens unit.

2. The ophthalmic device according to claim 1, wherein
a first optical path being an optical path of the first light includes an overlapped section that overlaps with a second optical path being an optical path of the second light, and a first nonoverlapped section that does not overlap with the second optical path,
the second optical path includes the overlapped section and a second nonoverlapped section that does not overlap with the first optical path,
the first objective lens unit is arranged in the first nonoverlapped section, and
the second objective lens unit is arranged in the second nonoverlapped section.

3. The ophthalmic device according to claim 2, wherein
the first scanning optical system comprises a first scanner configured to scan the first light outputted from the first light source,
the second scanning optical system comprises a second scanner configured to scan the second light outputted from the second light source,
the first scanner is arranged in the first nonoverlapped section, and
the second scanner is arranged in the second nonoverlapped section.

4. The ophthalmic device according to claim 2, further comprising:
a scanner configured to scan the first light outputted from the first light source and configured to scan the second light outputted from the second light source,
wherein
the scanner is arranged in the overlapped section and is shared by the first scanning optical system and the second scanning optical system,
the first objective lens unit is arranged between the scanner and the subject eye, and
the second objective lens unit is arranged between the scanner and the subject eye.

5. The ophthalmic device according to claim 4, further comprising:
a measuring window configured to face the subject eye; and
a first dichroic mirror configured to reflect the first light but allow the second light to penetrate,
wherein
the first range is an anterior segment of the subject eye,
the second range is a fundus of the subject eye,
the overlapped section includes a first overlapped section that includes a section connecting the subject eye and the measuring window, and
the first dichroic mirror is arranged at a first position where the first overlapped section branches into the first nonoverlapped section and the second nonoverlapped section.

6. The ophthalmic device according to claim 5, wherein a back surface of the first dichroic mirror comprises Anti-Reflection (AR) coating for a wavelength bandwidth of the first light.

7. The ophthalmic device according to claim 5 or 6, further comprising:
a second dichroic mirror configured to reflect the first light but allow the second light to penetrate,
wherein
the overlapped section includes a second overlapped section that includes a section connecting the scanner and a second position where the first nonoverlapped section and the second nonoverlapped section merge and branch, and
the second dichroic mirror is arranged at the second position.

8. The ophthalmic device according to claim 7, wherein a back surface of the second dichroic mirror comprises Anti-Reflection (AR) coating for a wavelength bandwidth of the first light.

9. The ophthalmic device according to claim 7 or 8, further comprising:
a third dichroic mirror arranged at a third position, which is a position at one end of the second overlapped section and different from the second position,
wherein the third dichroic mirror is configured to reflect the first light but allow the second light to penetrate.

10. The ophthalmic device according to claim 9, wherein a back surface of the third dichroic mirror comprises Anti-Reflection (AR) coating for a wavelength bandwidth of the first light.

11. An ophthalmic device comprising:
a first light source configured to output first light;
a second light source configured to output second light;
a first scanning optical system configured to scan the first light outputted from the first light source in a first range of a subject eye;
a second scanning optical system configured to scan the second light outputted from the second light source in a second range of the subject eye that is different from the first range;
a first interferometer configured to acquire tomographic information of the first range based on first interference light obtained from reflected light of the first light reflected on the subject eye;
a second interferometer configured to acquire tomographic information of the second range based on second interference light obtained from reflected light of the second light reflected on the subject eye;
a measuring window configured to face the subject eye; and
a first dichroic mirror configured to reflect the first light but allow the second light to penetrate,
wherein
the first range is an anterior segment of the subject eye,
the second range is a fundus of the subject eye,
a central wavelength of the first light and a central wavelength of the second light are different,
a first optical path being an optical path of the first light includes an overlapped section that overlaps with a second optical path being an optical path of the second light and a first nonoverlapped section that does not overlap with the second optical path,
the second optical path includes the overlapped section and a second nonoverlapped section that does not overlap with the first optical path,
the overlapped section includes a first overlapped section that includes a section connecting the subject eye and the measuring window,
the first dichroic mirror is arranged at a first position where the first overlapped section branches into the first nonoverlapped section and the second nonoverlapped section, and
a back surface of the first dichroic mirror comprises Anti-Reflection (AR) coating for a wavelength bandwidth of the first light.
